(19) Europäisches Patentamt

European Patent Office

Office européen des brevets

(11) Publication number: **0 280 561 B1**

# EUROPEAN PATENT SPECIFICATION

(45) Date of publication of patent specification: **22.09.93**

(51) Int. Cl.5: **G01N 33/546**, G01N 33/532, G01N 33/569, G01N 33/76

(21) Application number: **88301656.0**

(22) Date of filing: **26.02.88**

(54) **Agglutination reagent and method of preparing same.**

(30) Priority: **27.02.87 US 19850**
**18.09.87 US 98432**

(43) Date of publication of application:
**31.08.88 Bulletin 88/35**

(45) Publication of the grant of the patent:
**22.09.93 Bulletin 93/38**

(84) Designated Contracting States:
**CH DE FR GB LI**

(56) References cited:
**EP-A- 0 124 320**

**PATENT ABSTRACTS OF JAPAN, vol. 10, no. 282 (P-500)[2338], 25th September 1986; & JP-A-61 100 660 (MORINAGA MILK IND. CO., LTD) 19-05-1986**

**NATURE, vol. 210, 30th April 1986, pages 536-537; J. LENARD et al.: "Succinylation of gamma globulin"**

**Reagents for Organic Synthesis, Fieser & Fieser, john Wiley & Sons, New York, 1967 pages 664 and 1184**

(73) Proprietor: **EASTMAN KODAK COMPANY (a New Jersey corporation)**
**343 State Street**
**Rochester New York 14650(US)**

(72) Inventor: **Warren, Harold Chester, III EASTMAN KODAK COMPANY**
**Patent Department 343 State Street**
**Rochester New York 14650(US)**
Inventor: **Snyder, Brian Anthony c/o EASTMAN KODAK COMPANY**
**Patent Department 343 State Street**
**Rochester New York 14650(US)**
Inventor: **Belly, Robert Troconis c/o EASTMAN KODAK COMPANY**
**Patent Department 343 State Street**
**Rochester New York 14650(US)**

(74) Representative: **Nunney, Ronald Frederick Adolphe et al**
**Kodak Limited Patent Department Headstone Drive**
**Harrow Middlesex HA1 4TY (GB)**

## Description

This invention relates to an agglutination reagent and a method of preparing it. This reagent is useful in immunoassays and diagnostic analytical procedures.

The antigen-antibody reaction is the basis for all immunological test methods. Certain proteins known as antibodies are produced by mammals in response to the presence of an antigen, that is a foreign substance, which can be another protein or a carbohydrate. This normal body response to a foreign substance has led to the development of a number of techniques which are used to diagnose various diseases, disorders and physiological conditions. In a general sense, one component of an antibody-antigen reaction can be defined as the immunoreactive species while the corresponding component which complexes with it is considered the receptor.

In vitro tests for the presence of a suspected protein, antigen or antibody in a biological sample are carried out by adding the immunological counterpart to the biological sample. If the suspected substance is present, the resulting antigen-antibody reaction can be demonstrated by precipitation of the antigen-antibody complex. This reaction complex is generally difficult to detect visually. For this reason, either antibodies or antigens are often bound to insoluble particles, for example polymer latex particles, so that when the complex is formed, it is readily detectable from the resulting agglutination either by observing the presence of clumping or by a detectable tracer associated with the particles. Agglutination then is characterized by the clumping of particles from a suspension of particles. Further details of known agglutination methods are provided in U.S. Patents 4,419,453 and 4,459,361.

Biological samples which are assayed for various analytes may contain materials which cause nonspecific interactions of the immunoreactive species and the corresponding receptor. These nonspecific interactions may undesirably influence the assay results by showing false positives or by providing a high background so that a true positive result is difficult to detect. In addition, the polymeric particles to which immunoreactive species or receptors are attached can interact with each other due to surface charges. Moreover, the species or receptors attached to the particles can interact with each other as well, causing unwanted agglutination and inaccurate results.

Various methods have been devised to reduce nonspecific interactions, including controlling the assay pH and adding materials to modify attached proteins. One such method is described in U.S. Patent 4,591,571. This reference describes an agglutination reagent having antibodies adsorbed to carrier particles. Prior to attachment, the antibodies are chemically modified with an acylating agent. It is alleged that such treatment reduces nonspecific interactions.

When an immunoreactive species was prepared using the teaching of U.S. Patent 4,591,571, that is modifying the antibodies by succinylation before attachment to beads, no signal was obtained (see Example 6 below).

The problems noted above with the prior art reagents and methods are overcome with a method for preparing an agglutination reagent comprising covalently attaching an immunoreactive species to polymeric particles having a tracer material associated therewith,

the method characterized wherein the free primary and secondary amino groups of the attached immunoreactive species are chemically modified with an acylating, alkylating or sulfonylating agent.

An agglutination reagent comprises an immunoreactive species covalently attached to polymeric particles having a tracer material associated therewith,

the reagent characterized wherein the attached immunoreactive species has free primary and secondary amino groups which have been modified with an acylating, alkylating or sulfonylating agent.

The present invention provides an agglutination reagent which exhibits reduced nonspecific interactions, and which is highly sensitive to the analyte of interest. These advantages are achieved by first covalently attaching an immunoreactive species, which is a receptor for the analyte of interest, to polymeric particles, followed by chemical modification with an acylating, alkylating or sulfonylating agent to chemically modify primary and secondary amine groups. This is directly contrary to the teaching of U.S. Patent 4,591,571. It is shown in Example 6 below that a reagent prepared using the teaching of that patent is ineffective in a diagnostic assay whereas the reagent of the present invention is very useful in such an assay.

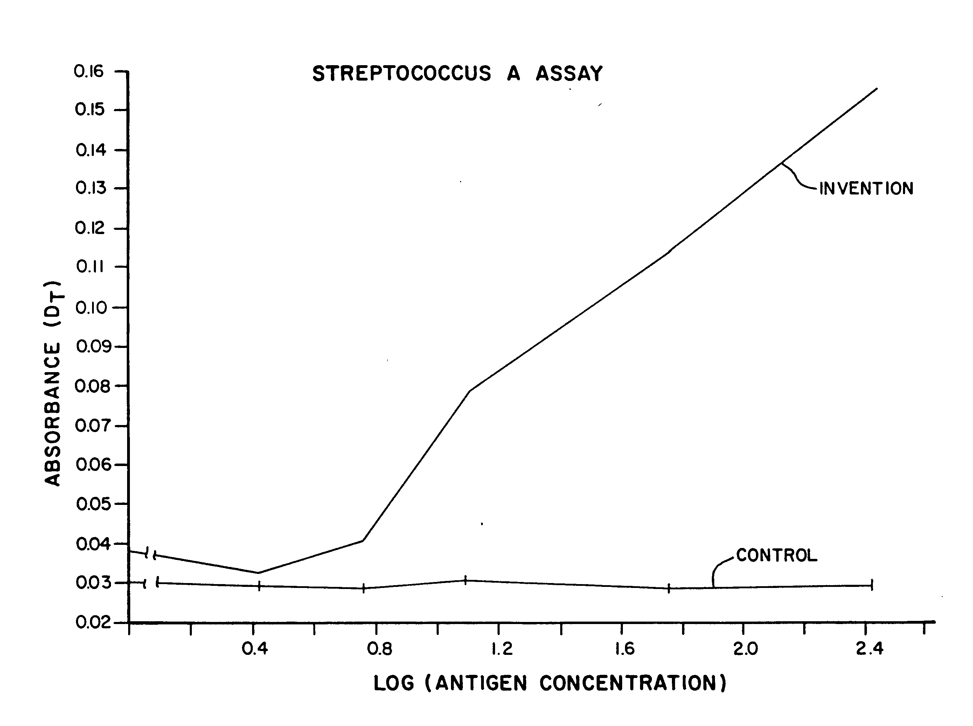

The Figure shows graphical plots of absorbance versus Streptococcus A antigen concentration for a reagent of the present invention as compared to a Control reagent prepared according to U.S. Patent 4,591,571. This comparison is described in detail in Example 6 below.

The present invention provides an agglutination reagent useful in a diagnostic test for an immunoreactive species (mono- or multivalent) which can be performed in a very short time, that is less than 10 minutes, and without the use of complicated equipment. This permits the test to be performed in a doctor's office or at home and enables the doctor or home user to know the results of the test very quickly. The test detects the presence of the species in a biological sample, such as a swab specimen from the throat, urine specimen or sample of another aqueous liquid. Such biological samples can be tested with or without pretreatment (for example, filtration) to remove unwanted debris or interferents.

The reagent of this invention can be used to detect and quantify any of a wide variety of immunoreactive species. For purposes of definition, the immunoreactive species to be determined is defined as a ligand herein. Such ligands are generally proteins, drugs, steroids, glycoproteins, glycolipids, carbohydrates or other biological or chemical compounds of interest which have one or more sites for complexing with a corresponding receptor, for example, corresponding antibodies for antigens. Alternatively, the ligand can be an antibody which has one or more complexing sites reactive with the corresponding antigen or an anti-antibody. Ligands which can be detected with the reagent of this invention include, but are not limited to, Streptococcus A antigen, antigens for chlamydial and gonococcal organisms, antigens from retroviruses such as HTLV and HIV (human immunodeficiency virus) or antibodies directed against such, human chorionic gonadotropin, leutinizing hormone, herpes viruses, drugs, antibiotics, and other hormonal, bacterial or viral antigens and antibodies. In some instances, the ligand must be extracted from the organism or virus found in the biological specimen. In other instances, the ligand is already in a reactive form and requires no extraction procedures prior to the assay. Extraction procedures for a given ligand are known to one skilled in the art. Exemplary extraction procedures for Streptococcus A antigen are described below. In still other instances, the ligand can be detected as part of an organism or virus without any extraction step.

Preferably, the reagent is used to detect Streptococcus A antigen as is demonstrated in the following embodiment and in Example 1 below. This embodiment relating to Streptococcus A antigen is presented for illustrative purposes, but it will be understood that the scope of this invention is not so limited. A biological sample suspected of containing the antigen can be collected from a patient in any suitable manner. Subsequently, if necessary, the antigens are extracted from the organisms in a suitable manner. A preferred extraction procedure includes dipping the swab in a suitable extraction composition containing one or more reagents which singly or in combination cause release of the Streptococcus A antigen from the organism, specimen cells and other debris in the sample.

Useful extraction compositions known in the art for Streptococcus A antigen include a mixture of nitrite salt and glacial acetic acid, as described in E.P. Publication 150,567, and enzymes derived from the bacterium Streptomyces albus as described in U.S. Patent 4,618,576. A preferred extraction composition is a mixture of a nitrite salt (for example, sodium nitrite or potassium nitrite) with an organic acid (for example, succinic, malonic or citric acid) with or without a binder material.

The presence of a ligand, for example Streptococcus A antigen, is detected using the agglutination reagent of this invention which comprises water-insoluble carrier particles having another immunoreactive species (which is a receptor for the ligand) covalently bound to the particles. Reaction (or immunochemical binding) between the ligand and receptor then results in a linking together of the particles so that they agglutinate and precipitate out of suspension. This agglutinate can be suitably detected using tracer materials associated with the particles.

Suitable particles useful in the reagent can be natural or synthetic particles which are water-insoluble and capable of having an immunoreactive species covalently bound thereto in a suitable manner. Examples of useful carrier particles include ferritin crystals, agarose particles, glass beads, polymeric particles, such as latex particles, and others known in the art which have reactive groups on the particle surface for covalent reaction with immunoreactive species. The following references describe representative useful particles: U.S. Patents 3,700,609, 3,853,987, 4,108,972, 4,401,765, 4,419,453, 4,459,361, 4,478,946 and 4,591,571. The particles useful in this invention are generally quite small, that is less than 2 micrometer in diameter. Preferably, they have an average diameter of from 0.1 to 1 micrometer.

The particles must have groups on the outer surfaces which can form covalent bonds with the immunoreactive species. Such groups include, but are not limited to, carboxyl, amine, epoxy, aldehyde, haloalkyl, activated 2-substituted ethylsulfonyl, vinylsulfonyl, vinylsulfonylalkylene and others known in the art. These groups can be incorporated into the particles in any suitable manner, for example, during manufacture, or just prior to attachment of the immunoreactive species. The haloalkyl, activated 2-substituted ethylsulfonyl, vinylsulfonyl and vinylsulfonylalkylene groups are preferred.

Particularly useful carrier particles are polymeric latex particles, and more preferably they are what are known in the art as core-shell polymeric latex particles. A wide variety of monomers can be used in the preparation of such particles as long as the particles are water-insoluble. A worker skilled in the polymer chemistry art would be able to design and prepare suitable latex particles. Preferred core-shell polymeric latex particles in the practice of this invention are described in Examples 1 and 3 below. These particles have a core composed of homo- or copolymers of styrene, or other monomers which have high affinity for a tracer material (described below) and a shell composed of homo- or copolymers which have the desired reactive groups free for reaction with the immunoreactive species.

The particles useful in the practice of this invention have sufficient tracer molecules associated therewith in order to allow quantitative determination of the ligand from the amount of tracer seen in either the agglutinate or in the unagglutinated residual materials. The tracer molecules can be suitably attached to the outer surface of the particles, or more preferably, distributed within the particles. Any tracer material which allows detection of the agglutinate can be used. If ferritin crystals are used as the particles, the tracer molecules are molecules of iron inherently in those crystals. Other natural or synthetic particles can have, as tracers: radioisotopes, colorimetric compounds, fluorescent compounds, bioluminescent compounds, chemiluminescent compounds, phosphorescent compounds and other detectable materials known in the art. Preferably, the tracer is a radioisotope, colorimetric compound or fluorescent compound (for example, a dye or rare earth chelate). A worker skilled in the art would be able to combine an appropriate tracer with the particular particle used.

In one embodiment, the tracer can be a fluorescent rare earth chelate such as an europium chelate, as described for example, in U.S. Patent 4,259,313. In another and preferred embodiment, the tracer is a colorimetric compound which is readily detected in the agglutinate. Useful dyes are known in the art. Some dyes can be incorporated into the particles when the particles are prepared. Alternatively, the dyes are imbided into preformed particles in such a manner that they do not leach out.

The tracer can be distributed within the particles in any suitable manner. For example, the tracer can be uniformly distributed therein as shown for example in U.S. Patent 3,853,987. Preferably, the tracer molecules are located in a restricted area of the particles, for example, near the surface or predominantly in the interior thereof. In the preferred core-shell particles, the tracer can be in either the core or shell, but most preferably, substantially all of it is in the core of the particles.

An immunoreactive species (that is, a receptor molecule reactive with the ligand) is covalently attached to the outer surfaces of the particles in a suitable manner using known procedures and reagents if needed. When the attached species is an antibody, either monoclonal or polyclonal antibodies can be used. Antibodies can be obtained commercially or prepared using known techniques. Either whole antibodies or fragments thereof may be used.

The attached immunoreactive species can be an antigen if the ligand is an antibody. One such reagent is an agglutination reagent useful for detecting antibodies to a human retrovirus antigen, for example, antibodies to HTLV-I or HIV-I antigen.

After attachment, the immunoreactive species is chemically modified with a suitable modifying agent which is capable of modifying primary and secondary amine groups. Examples of such modifying agents include acylating agents, alkylating and sulfonylating agents, some of which are described, for example, in Enzyme-Immunoassay, Maggio (Ed.), CRC Press, Inc., Boca Raton, Florida, 1980, pp. 72-77.

Representative useful acylating agents are described in U.S. Patent 4,591,571. Preferred acylating agents are selected from the group consisting of anhydrides, acyl halides and esters derived from dicarboxylic and polycarboxylic acids (three or more acid groups). The anhydrides, such as succinic anhydride, are most preferred. Representative alkylating and sulfonylating agents, such as bromoacetic acid, chloroacetic acid, fluoronitrobenzene, bromomalonic acid, bromopropionic acid, m-(chlorosulfonyl)-benzoic acid and p-(chlorosulfonyl)benzoic acid are also useful with bromoacetic acid being most preferred.

The chemical modification step of the method of this invention can be carried out generally by first covalently immobilizing the immunoreactive species onto the particles. Casein may also be immobilized thereon at the same time. The immobilized species is then modified with a suitable acylating, alkylating or sulfonylating agent under the appropriate conditions for a given agent. Such conditions are known to one skilled in the art. Representative procedures are described in Examples 1, 4 and 5 below.

In the modification process, the number of free primary and secondary amino groups of the immobilized immunoreactive species which are modified will depend upon the pH of the medium, the concentration of reagents and immunoreactive species. But a sufficient modification can be achieved by one skilled in the art using the teaching provided herein. Generally, the amount of modifying agent used is 10% of the amount of species present by weight.

Once an agglutinate has been formed in an assay, agglutinated materials are separated from unagglutinated materials in any suitable manner known in the art. Generally, the separation is accomplished with a filtration technique. Following separation, the amount of either the agglutinated or unagglutinated materials is determined using known procedures.

While the present invention is not so limited, the assay for a ligand can be carried out using a suitable test device which comprises a microporous membrane. Such a device can have one or more wells to which a specimen containing the ligand is added for reaction with the species on the surface of the agglutination reagent. The reagent can be added to the device during the assay, or incorporated therein at the time of manufacture. Once the agglutinate is formed, the unagglutinated residual materials can be washed through the membrane with the wash solution into a separate compartment below the membrane.

Example 1: Preparation and Use of an Agglutination Reagent for Streptococcus A Determination

This example demonstrates the preparation of an agglutination reagent of this invention and its use to determine Streptococcus A antigen.

Core-shell polymeric latex particles containing a red dye (Oil Red EGN) in the core were prepared by imbibing the dye into the particles that had been prepared using core/shell polymerization techniques. Dye was incorporated using the techniques described in Belgian Patent 843,647. The core of the particles was composed of poly(styrene-co-2-acetoacetoxyethyl methacrylate) (70:30 weight ratio) while the shell was composed of poly(m,p-chloromethylstyrene). The average diameter of the particles was about 0.45 micrometer. Monoclonal antibodies to Streptococcus A antigen and casein were covalently immobilized on these particles as follows: to 0.6 ml of 50 mmolar borate buffer (pH 8.5) was added to 0.1 mg of total protein comprised of a 10:1 mixture of anti-Strep A antibody (2.9 mg/ml solution in phosphate buffered saline solution, known in the art as PBS) and casein (10 mg/ml water). After mixing, 41.5 $\mu$l of a 5% suspension of the polymeric latex particles were added (to provide 0.3% solids) and the resulting solution was rotated (end-over-end) for 24 hours at 37°C to effect covalent attachment of the antibody and the casein to the particles to form an agglutination reagent.

A solution of succinic anhydride (10 mg/ml dimethyl sulfoxide) was added to a suspension of the agglutination reagent described above at a weight ratio of 1 part anhydride to 1 part total protein. The resulting suspension was mixed for four hours at 25°C, then centrifuged for 5 minutes at 7000 rpm and the resulting pellet was resuspended in 0.1 molar glycerine buffer (pH 8.5) to a concentration of 0.3% solids. This procedure chemically modified the primary and secondary amine groups of the proteins attached to the particles.

Streptococcus A antigen was extracted from an isolate obtained from a local hospital at 25°C for 1 minute using a solution of equal volumes of sodium nitrite (8 molar) and citric acid (0.2 molar). The solution was then neutralized with an equal volume of 3-(N-morpholino)propanesulfonic acid buffer (2 molar, pH 7.5) containing ethylenediaminetetraacetic acid (75 mmolar).

A nylon 66 microporous membrane (5 $\mu$m average pore size) was incorporated into a test well of a disposable test device.

A mixture of sodium chloride (80 $\mu$l, 1 molar), the agglutination reagent suspension described above (40 $\mu$l), and extracted antigen (80 $\mu$l) containing about $4.2 \times 10^5$ colony-forming units was added to the test well of the test device containing the membrane, and incubated therein for two minutes at 25°C. The fluid was then allowed to drain into a compartment below the membrane, and the agglutinate on the membrane was washed with 150 $\mu$l of a wash fluid having an ionic strength of 0.25.

After the washing step, the amount of dye in the agglutinate on the membrane was measured at 540 nm using reflectance measuring equipment. The Williams-Clapper transform (J. Optical Soc. Am., 43, p. 595, 1953) was used to calculate transmission density values. The agglutinate on the membrane was readily observable and had a significantly greater density value than the density of a background control (the difference was 0.148). These data indicate that the agglutination reagent of the present invention was useful for determination of Streptococcus A antigen from a biological sample.

Example 2: Determination of Gonorrhea

This example demonstrates the use of the agglutination reagent of the present invention for the determination of gonorrhea. The agglutination reagent used in this example was composed of latex particles comprised of poly(styrene-co-m,p-chloromethylstyrene-co-2-hydroxyethyl acrylate) (76:23:1 weight ratio) into which had been imbibed 5%, by weight, of europium (III) (thenoyltrifluoroacetone)₃ along with trioctylphosphine oxide in the ratio of 1 part chelate to 2 parts oxide according to the procedures described in Belgian Patent 843,647. The particles had an average diameter of about 0.45 micrometer.

Monoclonal antibodies to the PI antigen of the serogroup B of Neisseria gonorrhea (also known in the art as the PIB antigen) were covalently immobilized on the particles described above as follows: to 1.3 ml of 50 mM borate buffer (pH 8.5) was added 0.15 ml of 1.08 mg/ml antibody solution in phosphate buffered saline (PBS). In addition, 0.32 ml of a 1 mg/ml aqueous solution of casein was added in order to immobilize casein on the particles as well.

After mixing, 41.5 $\mu$l of a 5% suspension of the latex particles described above were added, and the resulting solution was mixed at 37°C for 24 hours. Succinic anhydride (0.174 ml of 10 mg/ml dimethyl sulfoxide solution) was added, and the resulting solution was mixed at 22°C for four hours in order to modify the amine groups of the attached proteins. This solution was then centrifuged for 10 minutes and the resulting pellet was resuspended in 0.1 molar glycine (pH 8.5) to give a mixture containing 0.3% solids of agglutination reagent.

The PIB antigen was extracted from a specimen of Neisseria gonorrhea using a mixture of 1% ethanolamine and 10 mM ethylenediaminetetraacetic acid, followed by sonication and filtration.

A nylon 66 microporous membrane having an average pore size of 5 micrometers was pretreated by dipping it into a 2% casein solution. A mixture of sodium chloride (50 $\mu$l, 6 molar), antigen solution (50 $\mu$l) having a specific amount of antigen (nanogram) and the agglutination indicator solution described above (50 $\mu$l) was added to a test tube, incubated at 22°C for 30 minutes, then filtered through the treated microporous membrane. The resulting agglutinate on the membrane was washed with 0.15 $\mu$l of 1 molar tricine buffer (pH 8.6). The amount of agglutinate was determined by measuring the amount of fluorescence in the agglutinate using standard surface fluorescence measuring equipment (excitation, 342 nm and emission, 610 nm). A Control solution containing specific amounts of an extract of a different antigen (that is, the PI antigen of the serogroup A of Neisseria gonorrhea, or also known as the PIA antigen) was treated in the same manner in order to measure nonspecific interactions with the antibodies to the PIB antigen. Table I below shows the results of these tests. It is clear that the assay of this invention can be used to determine a desired antigen of a specific serogroup of gonorrhea.

## T A B L E   I

| PIB Antigen Concentration (ng) | Relative Fluorescence | |
|---|---|---|
| | Test | Control |
| 100 | 107 | 32 |
| 10 | 332 | 120 |
| 1 | 248 | 73 |

Example 3: Assay for Human Chorionic Gonadotropin

This example demonstrates the practice of the present invention for the determination of human chorionic gonadotropin (hCG).

Core/shell polymeric particles were imbibed with Oil Red EGN dye according to known procedures. The particle cores were composed of poly(styrene-co-2-acetoacetoxyethyl methacrylate) (85:15 weight ratio), and the particle shells were composed of poly(m,p-chloromethylstyrene-co-methacrylic acid) (99.8:0.2 weight ratio). The particles had an average diameter of about 0.32 micrometer.

Monoclonal antibodies to two different epitopic sites of hCG and casein were covalently immobilized on these particles as follows: to 0.6 ml of 50 mmolar borate buffer (pH 8.5) were added 0.1 mg of 10:1 mixture of hCG antibody (2.9 mg/ml phosphate buffered saline solution) and casein (10 mg/ml water). After mixing, 41.5 $\mu$l of a 5% suspension of the latex particles described above were added and the resulting suspension was rotated (end-over-end) for 24 hours at 37°C to effect covalent attachment of the antibodies and casein to the particles to form an agglutination reagent.

A solution of succinic anhydride (10 mg/ml dimethyl sulfoxide) was added to a mixture of the agglutination reagent at a weight ratio of 1 part anhydride to 1 part total protein, and the resulting mixture was mixed for 4 hours at 25°C, centrifuged for 5 minutes at 7000 rpm in order to chemically modify the amine groups of the attached proteins. The resulting pellet was resuspended in 0.1 molar glycine (pH 8.5) to a concentration of 0.3% solids.

Various amounts of hCG (milli I.U./ml) were added to phosphate buffered saline solutions (0.1 molar sodium phosphate and 0.15 sodium chloride) containing 0.5% bovine serum albumin. A nylon 66 microporous membrane having an average pore size of about 5 micrometers was incorporated into a test well of a disposable test device similar to that described in Example 1 above. This membrane was washed with 2 drops of a 1% aqueous solution of succinylated casein. The hCG concentration in milli I.U. is defined as 5000 milli I.U. being equivalent to 1 microgram of purified hCG.

A mixture of 60 $\mu$l of 4 molar sodium chloride, 1 molar tricine buffer (pH 8.6), 60 $\mu$l of suspension of the agglutination reagent described above and 240 $\mu$l of the hCG solutions described above was added to test tubes, gently mixed and allowed to incubate at 25°C for 10 minutes. A portion of each solution (300 $\mu$l) was

added to the test well containing the membrane and allowed to flow through the membrane. Agglutinate formed on the membrane did not flow through, however. It was washed with 300 $\mu$l of a 1 molar sodium chloride solution, and the amount of dye in the agglutinate was measured at 540 nm as described in Example 1. The results of these measurements are shown in Table II below as transmission density ($D_T$). It indicates that the assay of this invention can be used to determine hCG.

### T A B L E    II

| hCG Antigen (milli I.U./ml) | $D_T$ |
|---|---|
| 0 | 0.043 |
| 500 | 0.047 |
| 1000 | 0.133 |

### Example 4:    Preparation of Agglutination Reagents Using Alkylating Reagents

Example 4: Preparation of Agglutination Reagents Using Alkylating Reagents

This example illustrates the modification of immobilized antibodies using three different alkylation reagents: chloroacetic acid, bromoacetic acid and bromopropionic acid, to prepare reagents of this invention.

Core-shell polymeric latex beads, comprising a core of poly(styrene-co-acetoacetoxyethyl methacrylate) (70:30 molar ratio) and a shell of poly(m&p-chloromethylstyrene), were prepared and imbibed with a 2.5% acetonitrile solution of Oil Red EGN dye using the procedure described in Example 1. The dyed beads were suspended in 0.05 molar sodium borate buffer (pH 8.5) containing 0.1% sodium azide to provide a suspension of 0.3% solids.

Monoclonal antibodies to Strep A antigen and casein were immobilized onto the beads as described in Example 1.

The immobilized antibody mixture (0.012 ml) was mixed with the appropriate alkylating reagent (0.012 ml of a 10 mg/ml dimethylsulfoxide solution) and heated at 37°C for 18 hours, then centrifuged at 7000 rpm for 5 minutes. The resulting pellet was suspended in 0.1 molar glycine buffer (pH 8.5) to provide the agglutination reagent as a 0.3% solid suspension.

Example 5: Agglutination Reagent Using Polymer Derived from A Chloromethylsulfonyl Monomer

Core-shell polymeric latex beads, comprising a core of poly(styrene-co-acetoacetoxyethyl methacrylate) (85:15 molar ratio) and a shell of poly[styrene-co-m & p-(2-chloroethylsulfonylmethyl)styrene] (95.5:4.5 molar ratio), were prepared and imbibed with a 3.5% acetonitrile solution of Oil Red EGN dye as described in Example 1. The beads were suspended in 0.05 molar sodium borate buffer (pH 8.5) containing 0.1% sodium azide to provide a 0.3% solid suspension.

Monoclonal antibodies to Strep A [0.026 ml of a 3.8 mg/ml solution in 0.05 molar borate buffer (pH 8.5) containing 0.1% sodium azide] and casein (0.01 ml of a 1 mg/ml solution in water) were mixed together in about 0.9 ml of borate buffer.

The beads (0.173 ml of a 1.73% buffer suspension) were added, and the mixture was mixed end-over-end for 24 hours at about 25°C. Succinic anhydride (0.01 ml of a 10 mg/ml of dimethylsulfoxide solution) was added, and the mixture was rocked for 3 hours. The mixture was centrifuged, the supernatant decanted, and the pellet was suspended in 0.1 normal glycine (pH 8.5) containing 0.1% sodium azide to provide a 0.3% solid suspension.

Example 6: Assay for Streptococcus A

This example shows an assay for Strep A comparing an agglutination reagent prepared by the method of the present invention to an agglutination reagent prepared by the method taught in U.S. Patent 4,591,571.

The agglutination reagent of the present invention was prepared and tested in a Strep A assay as follows:

7

Core-shell polymeric latex beads, comprising a core of poly(styrene-co-2-acetoacetoxyethyl methacrylate) (95:5 molar ratio) and a shell of poly(m&p-chloromethylstyrene-co-methacrylic acid), (98:2 molar ratio) were prepared and imbibed with a 2% acetonitrile solution of Oil Red EGN dye according to Example 1.

The dyed beads were suspended in 0.05 molar sodium borate buffer (pH 8.5) containing 0.1% sodium azide to provide a suspension of 0.3% solids.

Monoclonal antibodies to Strep A antigen and casein were covalently immobilized onto these beads, and modified by treatment with succinic anhydride as described in Example 1.

Strep A antigen was extracted from an isolate obtained from a local hospital at 25°C for one minute using a solution comprising 1.2 molar citric acid (10 $\mu$l) and 8 molar sodium nitrite (120 $\mu$l), then neutralized with 1 molar tricine, pH 8.5 (120 $\mu$l).

A nylon microporous membrane (5 $\mu$m Biodyne™ A) containing 1.07 g/m² of succinylated casein was incorporated into a disposable test device.

The bead composition (2 drops, about 90 $\mu$l) was added to the disposable device, followed by 1 drop (about 40 $\mu$l) of the extracted antigen. After 2 minutes, the solution was allowed to drain through the membrane, then a wash solution of 1 molar sodium chloride (2 drops, about 90 $\mu$l) was added. The dye on the membrane was then read by reflectance, and the values were converted to transmission density ($D_T$). A plot of the data, shown in the Figure, illustrates the improvement using our method.

A control agglutination reagent was prepared using the teachings of U.S. Patent 4,591,371 to acylate the antibodies prior to immobilization. Attempts to covalently attach the modified antibodies to the particles were made using similar conditions described above. The resulting reagents were tested in a Strep A assay.

More specifically, for the Control reagent, monoclonal antibodies to Strep A antigen were first modified by treatment with succinic anhydride as follows: the antibodies (0.33 mg) were dissolved in 0.05 molar sodium borate buffer, pH 8.5 (0.9 ml), containing 0.1% sodium azide, and a solution of succinic anhydride (0.33 ml of a 10 mg/ml) dimethylsulfoxide solution) was added. The mixture was then stirred for 3 hours at room temperature and then dialyzed against 0.05 molar borate buffer at 4°C for about 16 hours.

The succinylated antibodies (0.5 ml of a 0.33 mg/ml borate buffer solution) and a solution of casein (0.0087 ml of a 1 mg/ml water solution) were mixed in 0.5 molar borate buffer, pH 8.5 (0.7 ml), containing 0.1% sodium azide. The dyed core-shell bead suspension (0.174 ml) was then added to the antibody/casein mixture and rotated end-over-end at 37°C for 24 hours in an attempt to covalently attach the modified antibodies. The mixture was centrifuged, and the supernatant liquid decanted. The pellet was suspended in 0.1 molar glycine (pH 8.5) containing 0.1% sodium azide to provide a suspension of 0.3% solids.

A Control Strep A assay was run as described. A plot of the data is shown in the Figure. It is clear from the plotted data that very little agglutination was observed with the Control reagent. Apparently, modification of the antibodies prior to attachment does not allow a significant amount of covalent attachment of the antibodies.

**Claims**

1. A method for preparing an agglutination reagent comprising covalently attaching an immunoreactive species to polymeric particles having a tracer material associated therewith,
   the method characterized wherein the free primary and secondary amino groups of the attached immunoreactive species are chemically modified with an acylating, alkylating or sulfonylating agent.

2. An agglutination reagent comprising an immunoreactive species covalently attached to polymeric particles having a tracer material associated therewith,
   the reagent characterized wherein the attached immunoreactive species has free primary and secondary amino groups which have been modified with an acylating, alkylating or sulfonylating agent.

3. The reagent as claimed in claim 2 wherein the free primary and secondary amino groups of the attached immunoreactive species are modified with an acylating agent.

4. The reagent as claimed in claim 3 wherein the acylating agent is an anhydride, acyl halide or ester derived from a dicarboxylic or polycarboxylic acid.

5. The reagent as claimed in claim 4 wherein the acylating agent is succinic anhydride.

**6.** The reagent as claimed in any of claims 2 to 5 wherein the immunoreactive species is an antibody.

**7.** The reagent as claimed in claim 6 wherein the immunoreactive species is an antibody against any of: Streptococcus A antigen, human chorionic gonadotropin, a chlamydial antigen, a gonococcal antigen, a herpes virus or a human retrovirus.

**8.** The reagent as claimed in any of claims 2 to 5 wherein the immunoreactive species is an antigen.

**9.** The reagent as claimed in any of claims 2 to 8 wherein the immunoreactive species is attached to the particles through reactive haloalkyl, activated 2-substituted ethylsulfonyl, vinylsulfonyl or vinylsulfonylalkylene groups on the outer surface of the particles.

**10.** The reagent as claimed in any of claims 2 to 9 wherein the tracer material is a dye within the polymeric particles.

## Patentansprüche

**1.** Verfahren zur Herstellung eines Agglutinations-Reagens, bei dem man eine immunoreaktive Gattung an polymere Partikel mit einem assoziierten Tracer-Material kovalent bindet, dadurch gekennzeichnet, daß die freien primären und sekundären Aminogruppen der gebundenen immunoreaktiven Gattung chemisch mit einem Acylierungs-, Alkylierungs- oder Sulfonylierungsmittel modifiziert werden.

**2.** Agglutinations-Reagens mit einer immunoreaktiven Gattung, die kovalent an polymere Partikel gebunden ist, die mit einem Tracer-Material assoziiert sind, dadurch gekennzeichnet, daß die gebundene immunoreaktive Gattung freie primäre und sekundäre Aminogruppen aufweist, die modifiziert worden sind mit einem Acylierungs-, Alkylierungs- oder Sulfonylierungsmittel.

**3.** Reagens nach Anspruch 2, in dem die freien primären und sekundären Aminogruppen der gebundenen immunoreaktiven Gattung mit einem Acylierungsmittel modifiziert sind.

**4.** Reagens nach Anspruch 3, in dem das Acylierungsmittel ein Anhydrid, ein Acylhalogenid oder ein Ester, der sich von einer Dicarbonsäure oder Polycarbonsäure ableitet, ist.

**5.** Reagens nach Anspruch 4, in dem das Acylierungsmittel Bernsteinsäureanhydrid ist.

**6.** Reagens nach einem der Ansprüche 2 bis 5, in dem die immunoreaktive Gattung ein Antikörper ist.

**7.** Reagens nach Anspruch 6, in dem die immunoreaktive Gattung ein Antikörper für: Streptococcus-A-Antigen, menschliches chorionisches Gonadotropin, ein chlamydiales Antigen, ein gonococcales Antigen, ein Herpes-Virus oder ein menschliches Retrovirus ist.

**8.** Reagens nach einem der Ansprüche 2 bis 5, in dem die immunoreaktive Gattung ein Antigen ist.

**9.** Reagens nach einem der Ansprüche 2 bis 8, in dem die immunoreaktive Gattung an die Partikel über reaktive Haloalkyl-, aktivierte 2-substituierte Ethylsulfonyl-, Vinylsulfonyl- oder Vinylsulfonylalkylengruppen an die äußere Oberfläche der Partikel gebunden ist.

**10.** Reagens nach einem der Ansprüche 2 bis 9, in dem das Tracer-Material ein Farbstoff innerhalb der polymeren Partikel ist.

## Revendications

**1.** Procédé pour la préparation d'un réactif d'agglutination comprenant une espèce immunoréactive liée de façon covalente à des particules polymères associées à un traceur, le procédé étant caractérisé en ce que les groupes libres amino primaires ou secondaires de l'espèce immunoréactive liée sont modifiés chimiquement par un agent d'acylation, d'alkylation ou de sulfonation.

**2.** Réactif d'agglutination comprenant une espèce immunoréactive liée de façon covalente à des particules polymères associées à un traceur, le réactif étant caractérisé en ce que l'espèce immunoréactive liée comprend des groupes libres amino primaires ou secondaires qui ont été modifiés chimiquement par un agent d'acylation, d'alkylation ou de sulfonation.

**3.** Réactif selon la revendication 2 dans lequel les groupes libres amino primaires ou secondaires de l'espèce immunoréactive liée sont modifiés chimiquement avec un agent d'acylation

**4.** Réactif selon la revendication 3 dans lequel l'agent d'acylation est une anhydride, un halogénure acylé ou un ester d'acide carboxylique ou polycarboxylique.

**5.** Réactif selon la revendication 4 dans lequel l'agent d'acylation est l'anhydride succinique.

**6.** Réactif selon l'une quelconque des revendications 2 à 5 dans lequel l'espèce immunoréactive est un anticorps.

**7.** Réactif selon la revendication 6 dans lequel l'espèce immunoréactive est un anticorps contre : un antigène Streptocoque A, une gonadotrophine chorionique humaine, un antigène chlamyde, un antigène gonocoque, un virus de l'herpès ou un rétrovirus humain.

**8.** Réactif selon l'une quelconque des revendications 2 à 5 dans lequel l'espèce immunoréactive est un antigène.

**9.** Réactif selon l'une quelconque des revendications 2 à 8 dans lequel l'espèce immunoréactive est liée aux particules par l'intermédiaire de groupes haloalkyl réactifs, de groupes activés éthylsulfonyl substitués en position 2, de groupes vinylsulfonyl ou vinylsulfonylalkylène, situés sur la surface externe des particules.

**10.** Réactif selon l'une quelconque des revendications 2 à 9 dans lequel le traceur est un colorant placé dans les particules polymères.

STREPTOCOCCUS A ASSAY

ABSORBANCE ($D_T$) vs LOG (ANTIGEN CONCENTRATION)

INVENTION

CONTROL

EP 0 280 561 B1